# EUROPÄISCHE PATENTSCHRIFT

(11) **EP 0 852 335 B1**
(45) Veröffentlichungstag und Bekanntmachung des Hinweises auf die Patenterteilung: **28.08.2002**
(21) Anmeldenummer: 97121270.9
(22) Anmeldetag: 04.12.1997
(51) Int. Cl.: G01N 33/36

(54) **Verfahren und Vorrichtung zur Bestimmung der Klebeneigung von Baumwolle**
Method and apparatus for determining the tacking tendency of cotton
Méthode et appareil pour la détermination de la tendance à coller du coton

(30) Priorität: 07.12.1996 DE 19650945
(43) Veröffentlichungstag der Anmeldung: 08.07.1998
(73) Patentinhaber: DEUTSCHE INSTITUTE FÜR TEXTIL- UND FASERFORSCHUNG STUTTGART, 73770 Denkendorf (DE)
(72) Erfinder: Artzt, Peter Dr.-Ing, D-72766 Reutlingen (DE); Azarschab,Mehdi Dr, D-72770 Reutlingen (DE); Schmid, Hans-Peter, D-72765 Neuhausen (DE)
(74) Vertreter: Canzler, Rolf, Dipl.-Ing.

(56) Entgegenhaltungen:
- WO-A-95/22762
- FR-A- 2 691 545
- JP-A- 63 050 753

## Beschreibung

Die Erfindung betrifft ein Verfahren und eine Vorrichtung zur Bestimmung der Klebeneigung von Baumwolle. Bei der Verarbeitung von Baumwolle ist es ein seit langem bekanntes Problem, klebrige Fasern zu verarbeiten, da diese beim Verstrecken während des Spinnprozesses aneinander hängen bleiben und auch an den Druckwalzen der Streckwerke zu Wickelbildung neigen. Es gibt verschiedene Ursachen für dieses Kleben. Man spricht vom sogenannten "Honigtau", der sich auf den Fasern der geöffneten Kapseln ablagert. Dabei handelt es sich hier im wesentlichen um ein Sekret von Fliegen oder anderen Insekten, aber auch von Mikroorganismen, die die Baumwollpflanze befallen. Es gibt allerdings auch die Erscheinung, daß die Baumwolle erst bei ihrer Verarbeitung zum Kleben neigt. Dieses Kleben entsteht durch zerquetschte Trashpartikel beim Ginnen , d.h. beim Trennen der Baumwollfasern von den Samenkörnern.

Unabhängig davon, woher diese Klebeneigung rührt, ist die Wirkung jedoch die gleiche, daß es Schwierigkeiten bei der Verarbeitung derartiger Fasern im Spinnprozeß gibt. Es ist deshalb für den Spinner wichtig, schon beim Einkauf der Baumwolle feststellen zu können, ob eine Klebeneigung derselben besteht oder nicht. Nachdem nach der Ernte die verschiedensten Baumwollsorten untereinander gemischt werden und das Mischen auch in der Spinnerei Voraussetzung ist für eine gutes und dem Einsatzzweck entsprechendes Garn, besteht die Gefahr, daß zum Kleben neigende Baumwolle die gesamte Mischung verdirbt. Es ist deshalb besonders wichtig, am besten schon an der Rohbaumwolle festzustellen, ob Klebeneigung besteht, damit dieses Fasermaterial beizeiten ausgeschieden oder auch einer Sonderbehandlung unterzogen wird, wodurch dieses Kleben beseitigt wird, damit auf keinen Fall einwandfreie Baumwolle mit derartiger, zum Kleben neigender Baumwolle vermischt wird.

Gerade in jüngster Zeit ist das Problem der zum Kleben neigenden Baumwolle besonders stark in den Vordergrund getreten, nachdem die Schädlingsbekämpfung aus Kostengründen, aber auch aus Gründen der Ökologie immer mehr eingeschränkt wird. Es ist deshalb besonders wichtig, diese zum Kleben neigende Baumwolle frühzeitig zu erkennen, um zu verhindern, daß dieses befallene Fasermaterial mit einwandfreiem Fasermaterial vermischt wird.

Zum Erkennen und Bewerten der Klebrigkeit von Baumwolle sind verschiedene Verfahren bereits bekanntgeworden. Bei dem "Or cin"-Test wird beispielsweise mit einer chemischen Lösung ein Farbtest durchgeführt. Die so zu untersuchenden Baumwollproben werden im Soxhleth mit Methanol extrahiert. Der Methanol-Extrakt wird mit Wasser aufgenommen und durch Zugabe der schwefelsauren Orcin-Lösung je nach Kohlenhydratanteil angefärbt. Es ergeben sich bei geeigneter Lösungskonzentration Farbtöne von zitronengelb bis dunkelrot und braun, die zudem noch farbmetrisch erfaßt werden können. Die Auswertung erfolgt photometrisch. Die jeweilige Färbung ergibt sich aus der Gesamtzukkerkonzentration der Baumwoll-Probe.

Mit dieser Methode werden zwar im Analogieverfahren geeignete Aussagen über die Klebeneigung der Baumwolle erreicht, jedoch ist dieser Test recht aufwendig an Zeit und Apparaturen, insbesondere, wenn eine größere Menge von Baumwoll-Proben getestet werden soll.

Ein weiterer Nachteil ist, daß zwischen den photometrischen Ergebnissen und der betrieblichen Erfahrung mit klebriger Baumwolle jeweils Relationen hergestellt werden müssen, inwiefern ein bestimmtes photometrisches Ergebnis eine Verarbeitung zuläßt, bedingt zuläßt oder zu unerträglichen Schwierigkeiten führt.

Eine weitere Methode, die Klebrigkeit von Baumwolle festzustellen, ist das sog. "Thermodetectionsverfahren". Um Aufschluß über das zu erwartende, durch die Klebrigkeit beeinflusste Verarbeitungsverhalten in der Spinnerei zu bekommen, wird die Klebeneigung von Baumwolle an metallischen Oberflächen simuliert. Unter Einwirkung von Wärme und Druck auf die Probe wird die natürliche Klebeneigung verstärkt und somit eine längere Einwirkungszeit simuliert. Mit diesem Verfahren können sowohl Baumwollflocken aus dem Ballen als auch geöffnete Baumwollflocken und -faserbänder geprüft werden.

Nachteilig bei diesem Verfahren ist, daß eine aufwendige Probenvorbereitung erforderlich ist. Es muß ein gleichmäßiges Vlies im Format der Prüffläche geformt werden. Das Vlies wird auf eine Aluminiumfolie ausgebreitet. Es muß beachtet werden, daß die Fläche der Probe die Prüffläche zu mindestens 95 % abdeckt. Diese zwischen Aluminiumfolien ausgebreitete Probe wird schließlich auf eine Preßplatte aufgelegt und 12 Sekunden lang Druck und Wärme ausgesetzt. Unmittelbar nach Ablauf dieser Zeit muß die Wärmeplatte sofort angehoben werden. Anschließend erfolgt eine weitere zweiminütige Pressung mit der Nachpreßvorrichtung. Danach wird die Probe aus dem Gerät genommen und nach einer Abkühlzeit von mindestens 30 Minuten die obere Folie vorsichtig von der Probe abgelöst. Die nicht auf der Aluminiumfolie klebenden Fasern der Probe werden mit einem Wischer von Hand vorsichtig abgewischt. Die auf der Folie verbleibenden Klebepunkte mit anhaftenden Faserbüscheln werden gezählt. Auch für das Zählen müssen besondere Bedingungen eingehalten werden, um Zählfehler zu vermeiden. Es sind wenigstens drei Einzelprüfungen vorzunehmen. Wenn nicht mindestens zwei Ergebnisse in einer Klebrigkeitsklasse und das dritte Ergebnis in einer angrenzenden Klasse liegen, so ist die Anzahl der Prüfungen zu erhöhen, bis zwei Drittel der Prüfungsergebnisse einer Klebrigkeitsklasse zuzuordnen sind. Auch hier besteht der Nachteil, daß dieses Verfahren nicht nur aufwendig ist, sondern auch Relationen zwischen dem Zählergebnis und Erfahrungen der Verarbeitbarkeit hergestellt werden müssen.

Ein anderes, weniger aufwendiges Verfahren beinhaltet die Quetschung der Baumwolle zwischen zwei Walzen und die Zählung der Fasern, die dabei Klebenbleiben (WO 9522762).

Aufgabe der Erfindung ist es, ein Verfahren und eine Vorrichtung zu schaffen, die eine praxisgerechte Feststellung der Klebeneigung von Baumwolle gewährleistet.

Diese Aufgabe wird durch die Merkmale der Ansprüche 1 und 11 gelöst.

Dadurch, daß die Baumwoll-Proben entsprechend dem Spinnprozeß bis zur Einzelfaser aufbereitet und auf eine Prüffläche 41 abgelegt werden, ohne daß Wärmebehandlungen oder ähnliche dem Spinnprozeß fremde Maßnahmen angewandt werden, ist dieses Test-Verfahren außerordentlich praxisnah. Es findet eine Nachahmung der Spinnbedingungen statt und der sich dort zeigenden Auswirkungen, denn nur diese sind für den Spinnprozeß interessant. Es müssen keine Relationen und Analogien hergestellt werden zwischen Erscheinungen, die an sich mit dem Spinnprozeß selbst sonst nichts zu tun haben. Darüber hinaus ist das Verfahren einfach in seiner Handhabung und selbst die Auswertung kann automatisiert werden, so daß menschliche Einflüsse weitgehend ausgeschaltet sind.

Weitere Einzelheiten der Erfindung werden anhand der Zeichnungen erläutert.

Es zeigen
- Fig. 1 -: den Aufbau der Vorrichtung zur Durchführung des erfindungsgemäßen Verfahrens;
- Fig. 2 -: die Auflöseeinheit der Vorrichtung gemäß Figur 1;
- Fig. 3 -: die Zuführung der Fasern auf die Prüffläche 41 im Detail.

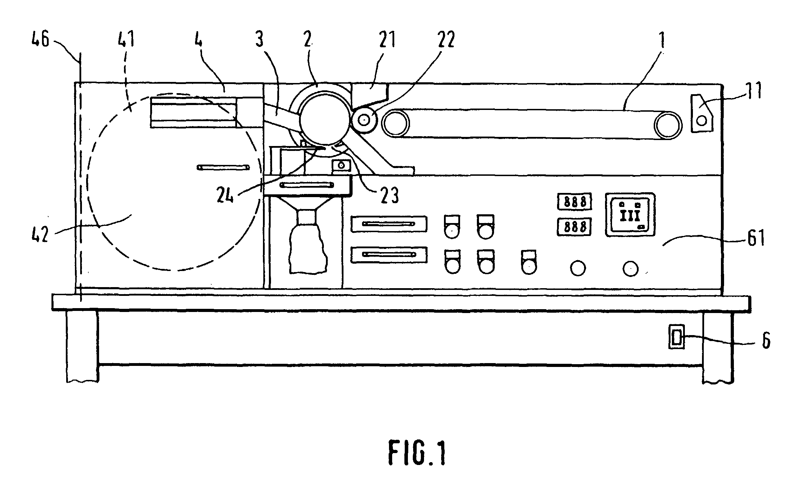

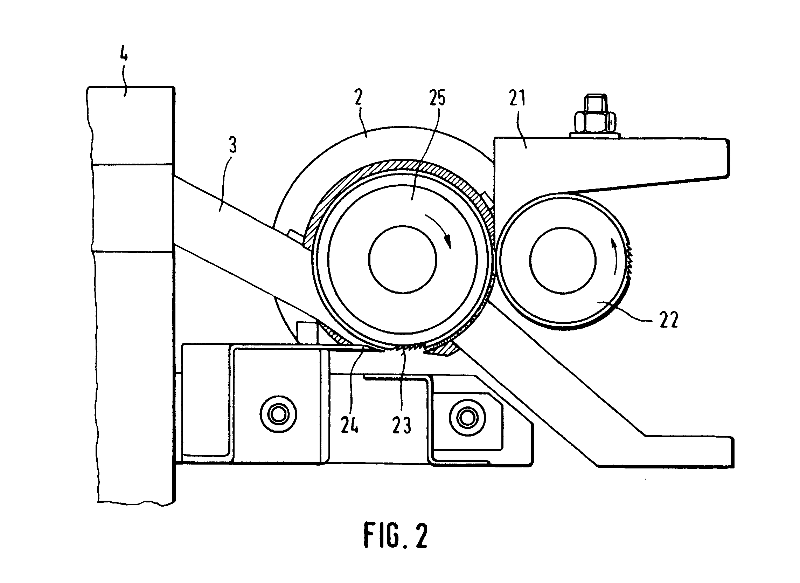

In Fig. 1 ist der Gesamtaufbau der Vorrichtung zur Bestimmung der Klebeneigung von Baumwolle schematisch dargestellt. Die Baumwoll-Probe wird auf das Transportband 1 gelegt und der Einzugswalze 22, die mit der Einzugsmulde 21 zusammenarbeitet, zugeführt. Durch den von der Einzugswalze 22 und der Einzugsmulde 21 gebildeten Klemmpunkt wird das Fasermaterial zurückgehalten, so daß die Öffnerwalze 25 (Fig. 2) der Öffnervorrichtung 2 Einzelfasern aus dem dargebotenen Faserbart herauslösen kann. Zur besseren Rückhaltung ist die Einzugswalze 22 mit einer Sägedrahtgarnitur bestückt, bei welcher die Zähne entgegen der Drehrichtung geneigt sind. Während die Öffnerwalze 25 mit hoher Drehzahl umläuft (ca. 8.000 U/min), ist die Einzugsgeschwindigkeit der Einzugswalze 22 gering (ca. 0,6 m/min), um eine gute Auflösung bis zur Einzelfaser zu erreichen.

Die aus dem Faserbart herausgelösten Fasern werden über eine Trash-Ausscheideöffnung 23 hinweg einem Faserkanal 3 zugeführt, durch welchen die Fasern in einem Luftstrom in die Prüfflächeneinheit 4 gelangen. Je nach Stellung des Trashmessers 24 kann eine Trash-Ausscheidung erfolgen oder auch unterbleiben.

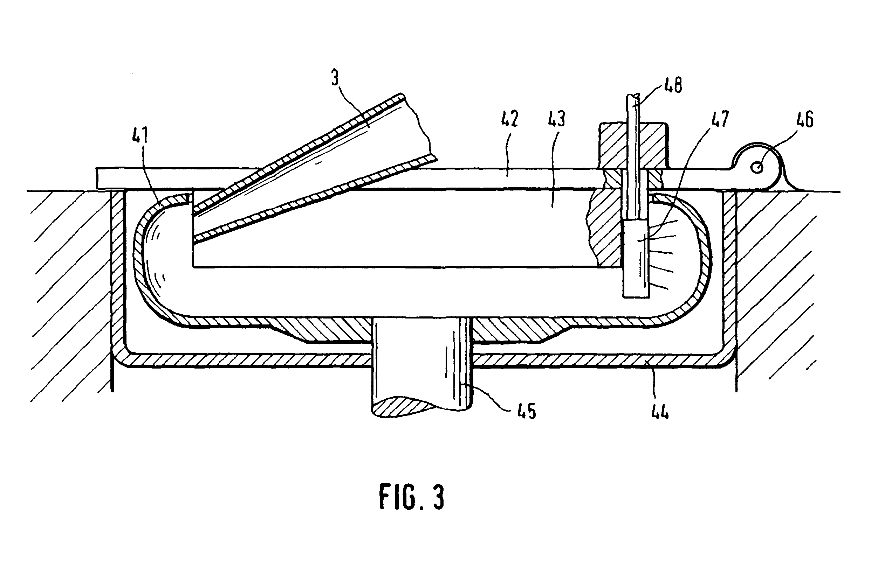

Die Prüfflächeneinheit 4 ist im einzelnen in Fig. 3 gezeigt. Die Prüffläche 41 selbst wird durch die Innenwand eines Rotors gebildet, der an dem Schaft 45 gelagert und über diesen angetrieben wird. Die Prüffläche 41 ist in einem Rotorgehäuse 44 untergebracht, das mit einem um das Scharnier 46 schwenkbaren Gehäusedeckel 42 verschließbar ist. Der Gehäusedeckel 42 weist einen Ansatz 43 auf, der bei geschlossenem Gehäusedeckel 42 in das Rotorinnere hineinragt und mit seiner Umfangsfläche der Prüffläche 41 gegenüber liegt. An der Umfangsfläche des Ansatzes 43 mündet der Faserkanal 3, durch welchen die Fasern von der Öffnereinheit 2 auf die Prüffläche 41 geleitet werden. Die als Rotor ausgebildete Prüffläche 41 ist zweckmäßig aus Kunststoff. Die mit den Fasern in Berührung kommende Prüffläche 41 ist so geglättet, daß die Fasern über diese Fläche gleiten können, ohne hängenzubleiben. Die Prüffläche 41 ist konkav gewölbt, so daß sich die durch den Faserkanal 3 eingespeisten Fasern zu einem Ring auf dieser Prüffläche 41 ansammeln. Die Prüffläche 41 muß groß genug gewählt werden, damit etwa 90 % der Fasern der zu untersuchenden Probe in Kontakt mit der Prüffläche 41 kommen.

Es hat sich nun als zweckmäßig erwiesen, daß die als Rotor ausgebildete Prüffläche 41 vorzugsweise einen Durchmesser von etwa 300 mm aufweist. Man erhält auf diese Weise bei Öffnung des Faserringes eine Bandlänge von 1 m. Die Breite der Prüffläche 41 beträgt etwa 6 cm, so daß sich die gesamte Prüffläche 41 auf 600 cm² bemißt. Beträgt das Probengewicht etwa 2 g/m, so werden die geforderten Bedingungen für die Prüffläche 41 von 600 cm² erfüllt.

Durch die Drehung des Rotors bei den o.g. Abmessungen mit etwa 4.000 U/min werden die gespeisten Fasern einer Fliehkraft ausgesetzt, die etwa das Zweitausendfache des Fasergewichtes beträgt. Durch diese Fliehkraft werden die Fasern gegen die Rotorwand als Prüffläche 41 gepreßt. Nach Einspeisung der gesamten Probenmenge wird der Rotor angehalten und der so entstandene Faserring aus dem Rotor herausgenommen. Nachdem die Prüffläche 41 so geglättet ist, daß Fasern ohne klebrigen Befall auf dieser nicht hängenbleiben, so ist der Rotor nach Herausnahme des Faserringes leer, es bleiben keine Fasern zurück. Handelt es sich jedoch um eine Probe, bei welcher die Fasern mit klebrigen Substanzen befallen sind, so bleiben diese nach Herausnahme des Faserringes auf der Prüffläche 41 haften. Die Menge der auf der Prüffläche 41 zurückgebliebenen Fasern ist dann ein Maß der Klebeneigung der Baumwolle. Um diesen für den Spinnprozeß relevanten Klebeeffekt an der Prüffläche zu erreichen, ist eine gewisse Mindestanpreßkraft erforderlich. Bei den verschiedensten Versuchen hat sich herausgestellt, daß bei den obengenannten Abmessungen der Prüffläche 41 zweckmäßig eine Anpreßkraft etwa von der Größe des Zweitausendfachen des Fasergewichtes benutzt wird, um die für den Spinnprozeß relevante Klebeneigung zu erfassen.

Wie bereits erwähnt, gibt die Menge der zurückgebliebenen Fasern Aufschluß über die Klebeneigung der Baumwolle. Diese Menge kann sowohl gewichtsmäßig als auch durch Zählen ermittelt werden. Die zahlenmäßige Erfassung erfolgt durch Auszählen der Klebepunkte, was sowohl von Hand als auch automatisch erfolgen kann. In Fig. 3 ist an dem Ansatz 43 gegenüber der Prüffläche 41 ein Sensor 47 angeordnet, mit dessen Hilfe die sich hell auf der schwarzen Prüffläche 41 abhebenden Fasern gezählt werden. Derartige Sensorzähleinrichtungen sind bekannt, so daß sie hier nicht weiter beschrieben werden müssen. Der Sensor 47 ist an einer Haltestange 48 beweglich gelagert und kann somit in den Ansatz 43 zurückgezogen werden, damit der Aufspeisvorgang nicht gestört wird. Andererseits kann der Sensor 47 in die für das Zählen optimale Lage gegenüber der Prüffläche 41 gebracht werden. Der Sensor 47 kann direkt mit einer Auswerteinrichtung verbunden sein, die die Zählergebnisse auswertet.

Bei der gewichtsmäßigen Ermittlung der klebenden Fasermenge werden nach dem Herausnehmen des Faserringes die zurückbleibenden Fasern von der Prüffläche abgestreift und gewogen. Das Verhältnis des Gewichtes der klebenden Fasern zum Probengewicht in % ist die Maßgröße für die Klebeneigung.

Bekanntlich hat das Klima einen Einfluß auf die Klebeneigung der Baumwolle während des Spinnverfahrens. Verändert man beispielsweise das Klima bezüglich Temperatur oder Feuchtigkeit bei dem oben beschriebenen Prüfverfahren, so läßt sich unschwer auch feststellen, ob beispielsweise eine Baumwolle durch Änderung der klimatischen Bedingungen in ihrer Klebeneigung beeinflußt werden kann. Es kann sich dabei herausstellen, daß eine Baumwolle, die sich zum Verspinnen bei normalem Spinnklima als ungeeignet erwiesen hat, durch Veränderung der klimatischen Bedingungen doch noch durchaus als verspinnbar befunden wird. Das Besondere dieses erfindungsgemäßen Verfahrens liegt eben darin, daß praktisch der Spinnprozeß nachgeahmt wird und somit eine direkte Beziehung zwischen den Ergebnissen der Prüfung und den tatsächlichen Spinnverhältnissen besteht. Es müssen keinerlei Analogien zwischen Testergebnissen und Erfahrungswerten erst hergestellt werden.

Mit dem erfindungsgemäßen Verfahren können auch Baumwoll-Proben direkt vom Feld entnommen geprüft werden, so daß rechtzeitig eine Vermischung mit nichtklebender Baumwolle verhindert werden kann. Hier ist es allerdings notwendig, vorher auf einer Labor-Gin die Baumwollfasern von den Samenkörnern zu befreien und die so gewonnenen Proben auf das Zuführband zu legen. Es können auch Faserbänder getestet werden, die unmittelbar auf das Transportband angelegt werden. Hierfür ist eine Bandführung 11 zusätzlich vorgesehen.

Eine weitere Abwandlung des erfindungsgemäßen Verfahrens besteht darin, daß bei der Öffnung durch die Trash-Abscheideöffnung 23 und das Trashmesser 24 Trash ausgeschieden wird. Sollte nämlich die Klebeneigung auf beim Ginnen zerquetschte Samenkörner zurückzuführen sein, so werden dann die klebenden Trashpartikel ausgeschieden. Es kann somit speziell nachgewiesen werden, ob die Klebeneigung tatsächlich nur von den Fasern herrührt. Aufgrund dieses Testes können so entsprechende Maßnahmen ergriffen werden, um das Zerquetschen der Samenkörner beim Ginnen zu verhindern.

Die Einfachheit und direkte Analogie des Verfahrens ermöglicht es, Baumwollfasermaterial, sei es als Rohbaumwolle vor dem Ginnen oder auch aus dem Ballen, als Flocke oder als Faserband schnell und unproblematisch zu prüfen. Durch die automatisierte Auswertung werden auch menschliche Unzulänglichkeiten bei der Auswertung vermieden.

### Bezugszeichenliste

- 1: Zuführband (Transportband)
- 11: Bandführung
- 2: Öffnervorrichtung
- 21: Einzugsmulde
- 22: Einzugswalze
- 23: Trash-Ausscheideöffnung
- 24: Trashmesser
- 3: Faserkanal
- 4: Prüfflächeneinheit
- 41: Prüffläche
- 42: Gehäusedeckel
- 43: Ansatz
- 44: Rotorgehäuse
- 45: Rotorschaft
- 46: Scharnier
- 47: Sensor
- 48: Haltestange
- 6: Schalter
- 61: Bedienungstafel

## Patentansprüche

1. Verfahren zur Bestimmung der Klebeneigung von Baumwolle anhand von Baumwollproben, **dadurch gekennzeichnet, daß** die dem zu prüfenden Material entnommene Probe entsprechend dem Spinnprozeß bis zur Einzelfaser aufbereitet und die so erhaltenen Fasern unter Einwirkung einer bestimmten Fliehkraft als Faserverband auf einer Prüffläche (41) abgelegt werden, daß nach vollständiger Aufspeisung der Probe der abgelegte Faserverband abgenommen und die auf der Prüffläche (41) hängengebliebenen Fasern nach ihrer Menge bestimmt werden.

2. Verfahren nach Anspruch 1, **dadurch gekennzeichnet, daß** die von einem Baumwollballen entnommene Probe einer Öffnervorrichtung (2) zugeführt und über einen Faserkanal (3) in einem Luftstrom einer rotierenden Prüffläche (41) zugeführt wird.

3. Verfahren nach Anspruch 2, **dadurch gekennzeichnet, daß** die von Rohbaumwolle entnommene Probe zuerst über eine Labor-Gin-Einrichtung zur Entfernung der Samenkerne geleitet wird.

4. Verfahren nach einem oder mehreren der Ansprüche 1 bis 3, **dadurch gekennzeichnet, daß** die Fasern auf der Prüffläche (41) einer Fliehkraft von etwa dem Zweitausendfachen des Fasergewichtes ausgesetzt werden.

5. Verfahren nach einem oder mehreren der Ansprüche 1 bis 4, **dadurch gekennzeichnet, daß** die Prüffläche (41) vorzugsweise einen Durchmesser von etwa 300 mm aufweist und die Ablage unter einer Rotationsgeschwindigkeit von 4.000 U/min. erfolgt.

6. Verfahren nach einem oder mehreren der Ansprüche 1 bis 5, **dadurch gekennzeichnet, daß** die Menge der klebenden Fasern in % des Probengewichtes ermittelt wird.

7. Verfahren nach einem oder mehreren der Ansprüche 1 bis 6, **dadurch gekennzeichnet, daß** die Klebeneigung nach der Anzahl der Klebepunkte bestimmt wird.

8. Verfahren nach Anspruch 7, **dadurch gekennzeichnet, daß** das Zählen der nach dem Herausnehmen des Faserverbandes auf der Prüffläche (41) verbleibenden Fasern mittels eines Sensors (47) im Rotor automatisch erfolgt und einem Auswertgerät zugeführt wird.

9. Verfahren nach einem oder mehreren der Ansprüche 1 bis 8, **dadurch gekennzeichnet, daß** für die Bestimmung der Klebeneigung eine Probe vom Gewicht von ca. 2 g/m genommen wird.

10. Verfahren nach einem oder mehreren der Ansprüche 1 bis 9, **dadurch gekennzeichnet, daß** die Prüffläche (41) so groß gewählt wird, daß etwa 90 % der Fasern in Kontakt mit der Prüffläche (41) kommen.

11. Verfahren nach einem oder mehreren der Ansprüche 1 bis 10, **dadurch gekennzeichnet, daß** die Durchführung des Prüfverfahrens unter verschiedenen klimatischen Bedingungen erfolgt.

12. Verfahren nach einem der Ansprüche 1 bis 11, **dadurch gekennzeichnet, daß** vor Ablage der Fasern auf der Prüffläche (41) Trash ausgeschieden wird.

13. Vorrichtung zur Bestimmung der Klebeneigung von Baumwolle anhand von Baumwollproben, die dem zu prüfenden Fasermaterial entnommen werden, mit einer Zuführvorrichtung zum Zuführen der Baumwollproben, **gekennzeichnet durch** eine Öffnervorrichtung, die aus einer Einzugswalze (22) und einer Einzugsmulde (21) sowie einer Öffnerwalze (25) besteht, ferner **gekennzeichnet durch** einen Faserkanal (3) und einer am Ende des Faserkanals angeordneten als Rotor ausgebildeten Prüffläche (41).

14. Vorrichtung nach Anspruch 13, **dadurch gekennzeichnet, daß** dem Zuführband (1) eine Gin-Vorrichtung vorgeschaltet ist.

15. Vorrichtung nach einem der Ansprüche 13 und 14, **dadurch gekennzeichnet, daß** die Innenoberfläche des Rotors einen Umfang von 100 cm und eine Breite von 6 cm aufweist.

16. Vorrichtung nach einem oder mehreren der Ansprüche 13 bis 15, **dadurch gekennzeichnet, daß** ein Zählsensor (47) der Prüffläche (41) zugeordnet ist.

17. Vorrichtung nach Anspruch 16, **dadurch gekennzeichnet, daß** der Zählsensor (47) mit einer Auswertvorrichtung verbunden ist.

18. Vorrichtung nach einem oder mehreren der Ansprüche 13 bis 17, **dadurch gekennzeichnet, daß** der Rotor aus Kunststoff besteht.

## Claims

1. A method for determining the adhesion tendency of cotton by using cotton samples, **characterized in that** the sample taken from the material to be tested is opened in accordance with the spinning process until it disintegrates into individual fibres and the fibres thus obtained are deposited, as a fibre formation, on a testing surface (41) while being exposed to a certain centrifugal force and that the deposited fibre formation, following a complete disintegration of the sample, is taken off and the fibres which have stuck to the testing surface (41) are determined as to their quantity.

2. The method according to claim 1, **characterized in that** the sample withdrawn from a cotton bale is fed to an opener device (2) and is introduced into an air stream of a rotary testing surface (41) through a fibre duct (3).

3. The method according to claim 2, **characterized in that** the sample withdrawn from natural cotton is first passed over a laboratory-scale gin device to remove the seeds therefrom.

4. The method according to one or more of claims 1 to 3, **characterized in that** the fibres located on the testing surface (41) are exposed to a centrifugal force of approximately two thousand times the fibre weight.

5. The method according to one or more of claims 1 to 4, **characterized in that** the testing surface (41) is preferably of a diameter of about 300 mm and depositing is effected under a rotational speed of 4,000 r.p.m.

6. The method according to one or more of claims 1 to 5, **characterized in that** the quantity of the adhering fibres is determined in percentage of the sample weight.

7. The method according to one or more of claims 1 to 5, **characterized in that** the adhesion tendency is determined according to the number of adhesion points.

8. The method according to claim 7, **characterized in that** the fibres remaining on the testing surface (41) after the removal of the fibre formation are automatically counted by means of a sensor (47) in the rotor and the count is fed to an evaluating apparatus.

9. The method according to one or more of claims 1 to 8, **characterized in that** a sample of abt. 2 g/m is withdrawn from the weight to determine the adhesion tendency.

10. The method according to one or more of claims 1 to 9, **characterized in that** the testing surface (41) is chosen to be so large that about 90 % of the fibres get into a contact with the testing surface (41).

11. The method according to one or more of claims 1 to 10, **characterized in that** the testing method is carried out under various climatic conditions.

12. The method according to any one of claims 1 to 11, **characterized in that** trash is separated out before the fibres are deposited on the testing surface (41).

13. An apparatus for determining the adhesion tendency of cotton by using cotton samples withdrawn from the fibre material under testing, including a feeding device for feeding the cotton samples, **characterized by** an opener device comprised of an intake roller (22) and a feed plate (21) as well as an opener roller (25), further **characterized by** a fibre duct (3) and a testing surface (41) disposed at the end of the fibre duct and formed as a rotor.

14. The apparatus according to claim 13, **characterized in that** the feeding belt (1) is preceded by a gin device.

15. The apparatus according to any one of claims 13 and 14, **characterized in that** the inside surface of the rotor has a circumference of 100 cm and a width of 6 cm.

16. The apparatus according to one or more of claims 13 to 15, **characterized in that** the testing surface (41) has associated therewith a counter sensor (47).

17. The apparatus according to claim 16, **characterized in that** the counter sensor (47) is coupled to an evaluation device.

18. The apparatus according to one or more of claims 13 to 17, **characterized in that** the rotor is made of a plastic.

## Revendications

1. Procédé pour déterminer la tendance d'adhésion de coton à l'aide d'échantillons de coton, **caractérisé en ce que** l'échantillon prélevé sur la matière à contrôler est traité comme dans le processus de filage jusqu'aux fibres et **en ce que** les fibres ainsi obtenues sont déposées, comme assemblage de fibres, sur une surface de contrôle (41) sous l'effet d'une force centrifuge déterminée, **en ce qu'**après le dépôt complet de l'échantillon, l'assemblage de fibres déposé est enlevé pour déterminer la quantité des fibres restées attachées sur la surface de contrôle (41).

2. Procédé selon la revendication 1, **caractérisé en ce que** l'échantillon prélevé sur une balle de coton est amené vers un dispositif d'ouvraison (2) et qu'il est acheminé dans un courant d'air vers une surface de contrôle (41) rotative, par l'intermédiaire d'un canal à fibres (3).

3. Procédé selon la revendication 2, **caractérisé en ce que** l'échantillon prélevé de coton brut doit d'abord passer une installation de nettoyage des grains dans le laboratoire pour que les grains soient éliminés.

4. Procédé selon l'une ou plusieurs des revendications 1 à 3, **caractérisé en ce que** les fibres sont exposées sur la surface de contrôle (41) à une force centrifuge qui est environ deux mille fois supérieure au poids des fibres.

5. Procédé selon l'une ou plusieurs des revendications 1 à 4, **caractérisé en ce que** la surface de contrôle (41) présente de préférence un diamètre d'environ 300 mm et que le dépôt est effectué à une vitesse de rotation de 4.000 trs/min.

6. Procédé selon l'une ou plusieurs des revendications 1 à 5, **caractérisé en ce que** la quantité des fibres adhésives est déterminée en pour-cent du poids de l'échantillon.

7. Procédé selon l'une ou plusieurs des revendications 1 à 6, **caractérisé en ce que** la tendance d'adhésion est déterminée en fonction du nombre de points d'adhésion.

8. Procédé selon la revendication 7, **caractérisé en ce que** le comptage des fibres restées sur la surface de contrôle (41) après enlèvement de l'assemblage de fibres est effectué automatiquement à l'aide d'un palpeur dans le rotor pour être transmis ensuite à un appareil d'évaluation.

9. Procédé selon l'une ou plusieurs des revendications 1 à 8, **caractérisé en ce que** pour la détermination de la tendance d'adhésion il est prélevé un échantillon d'un poids d'environ 2 g/m.

10. Procédé selon l'une ou plusieurs des revendications 1 à 9, **caractérisé en ce que** la surface de contrôle (41) est choisie en de telles dimensions qu'environ 90 % des fibres viennent en contact avec la surface de contrôle (41).

11. Procédé selon l'une ou plusieurs des revendications 1 à 10, **caractérisé en ce que** la mise en oeuvre du procédé de contrôle est effectuée dans des conditions climatique différentes.

12. Procédé selon l'une des revendications 1 à 11, **caractérisé en ce que** de l'ampasite est écartée avant le dépôt des fibres sur la surface de contrôle (41).

13. Dispositif pour déterminer la tendance d'adhésion de coton, à l'aide d'échantillons de coton prélevés sur la matière de fibres à contrôler, et qui comporte un dispositif d'amenée pour l'amenée des échantillons de coton, **caractérisé par** un dispositif d'ouvraison qui est constitué par un cylindre d'alimentation (22) et une auge d'alimentation (21) ainsi que d'un cylindre ouvreur (25), et **caractérisé en plus de cela** par un canal à fibres (3) et une surface de contrôle (41) conformée comme rotor et disposée au bout du canal à fibres.

14. Dispositif selon la revendication 13, **caractérisé en ce qu'**en amont de la bande d'alimentation il est disposé une installation de nettoyage des grains.

15. Dispositif selon l'une des revendications 13 et 14, **caractérisé en ce que** la surface intérieure du rotor présente une circonférence de 100 cm et une largeur de 6 cm.

16. Dispositif selon l'une ou plusieurs des revendications 13 à 15, **caractérisé en ce qu'**un palpeur de comptage (47) est affecté à la surface de contrôle (41).

17. Dispositif selon la revendication 16, **caractérisé en ce que** le palpeur de comptage (47) est relié à une installation d'évaluation.

18. Dispositif selon l'une ou plusieurs des revendications 13 à 17, **caractérisé en ce que** le rotor consiste en une matière artificielle.
